(19) Europäisches Patentamt
European Patent Office
Office européen des brevets

(11) Veröffentlichungsnummer: **0 143 303**
**A2**

(12) # EUROPÄISCHE PATENTANMELDUNG

(21) Anmeldenummer: **84112433.2**

(22) Anmeldetag: **16.10.84**

(51) Int. Cl.[4]: **C 07 C 103/147**, C 07 C 102/00

(30) Priorität: **29.10.83 DE 3339386**
**30.05.84 DE 3420112**

(43) Veröffentlichungstag der Anmeldung: **05.06.85**
**Patentblatt 85/23**

(84) Benannte Vertragsstaaten: **CH DE FR GB IT LI**

(71) Anmelder: **BAYER AG, Konzernverwaltung RP**
**Patentabteilung, D-5090 Leverkusen 1 Bayerwerk (DE)**

(72) Erfinder: **Kadelka, Jürgen, Dr.,**
**Bodelschwinghstrasse 12, D-4150 Krefeld 1 (DE)**
Erfinder: **Schwarz, Hans-Helmut, Dr., Ratherstrasse 90,**
**D-4150 Krefeld 1 (DE)**

(54) **Verfahren zur Herstellung von substituierten Bernsteinsäureamiden.**

(57) Substituierte Bernsteinsäureamide, insbesondere unterschiedlich derivatisierte substituierte Bernsteinsäureamide, werden hergestellt durch Umsetzung ungesättigter Carbonsäureamide mit Kohlenmonoxid und mit einer nukleophilen Komponente mit mindestens einem beweglichen Wasserstoffatom in Gegenwart von Kobaltverbindungen und gegebenenfalls in Gegenwart von einer oder mehreren tertiären Stickstoffbasen bei erhöhtem Druck und erhöhter Temperatur.

BAYER AKTIENGESELLSCHAFT 5090 Leverkusen, Bayerwerk

Konzernverwaltung RP Bg/by-c
Patentabteilung

# Verfahren zur Herstellung von substituierten Bernsteinsäureamiden

Die Erfindung betrifft ein Verfahren zur Herstellung von substituierten Bernsteinsäureamiden, insbesondere zur Herstellung von unterschiedlich derivatisierten, substituierten Bernsteinsäureamiden durch Umsetzung geeigneter Carbonsäureamide mit Kohlenmonoxid und mit einer nukleophilen Komponente mit mindestens einem beweglichen Wasserstoffatom in Gegenwart von Kobaltverbindungen und gegebenenfalls in Gegenwart einer oder mehrerer tertiärer Stickstoffbasen bei erhöhtem Druck und erhöhter Temperatur.

Aus J. Chem. Soc. 1953, 3490 ist bekannt, daß 2-substituierte Bernsteinsäureester-anilide ausgehend von entsprechend substituierten N-Phenylbernsteinsäureimiden durch hydrolytische Spaltung und anschließende Veresterung erhalten werden. Diese Methode ist aufwendig, da sie mehrere Reaktionsschritte beinhaltet. Zudem bereitet die Trennung der durch Hydrolyse erhaltenen isomeren Halbderivate große Schwierigkeiten. Je nachdem welches Isomer synthetisiert werden soll, sind darüber hinaus die Ausbeuten niedrig.

Eine andere Methode, z.B. 2-Methylbernsteinsäure-1-ester-4-amid herzustellen, wird in J. Amer. Chem. Soc. 81, 4946 (1959) beschrieben. Die Hydrierung von ß-Carboxycrotonsäureamid liefert das Halbamid der Methylbernsteinsäure, dessen anschließende Veresterung führt zum gewünschten Reaktionsprodukt. Nachteilig ist hierbei die Anzahl der Reaktionsschritte sowie die Zugänglichkeit der als Ausgangsstoffe eingesetzten ungesättigten Carbonsäurederivate.

Ferner ist bekannt, daß man durch Umsetzung von Olefinen mit Kohlenmonoxid und einer H-aciden Komponente, wie Wasser, Alkohol oder Amin, in Gegenwart eines Katalysators, der ein Metall der 8. Nebengruppe des Periodensystems der Elemente enthält, Carbonsäuren bzw. Carbonsäurederivate herstellen kann (J. Falbe, Synthesen mit Kohlenmonoxid, Springer-Verlag, Berlin, Heidelberg, New York 1967).

Die Hydrocarboxylierung von Crotonsäure in Gegenwart von Kobaltcarbonyl in Aceton als Lösungsmittel liefert ein nicht näher quantifiziertes Gemisch von Glutar- und Methylbernsteinsäure (Chim. Ind. 37, 1029 (1955); CA Vol. 50, 11 239). Die erzielten Ausbeuten sind nur mäßig; zudem besitzen beide Komponenten des Produktgemisches jeweils die gleiche funktionelle Endgruppe.

Gemischt funktionalisierte Dicarbonsäurediester lassen sich gemäß EP-OS 80 957 in Gegenwart von Kobaltkatalysatoren und tertiären aromatischen Stickstoffbasen

durch Umsetzung α,ß-ungesättigter Carbonsäureester mit Kohlenmonoxid und Alkohol synthetisieren. Diese Methode führt jedoch zu linearen Dicarbonsäurederivaten.

Es wurde nun ein Verfahren zur Herstellung von substituierten Bernsteinsäureamiden der Formel

$$R^4-\overset{O}{\overset{\|}{C}}-\underset{R^3}{\underset{|}{CH}}-CH_2-\overset{O}{\overset{\|}{C}}-NR^1R^2 \qquad (I),$$

worin

$R^1$ und $R^2$ gleich oder verschieden sein können und für Wasserstoff, einen Alkyl- oder Cycloalkyl-Rest mit 1 bis 20 Kohlenstoffatomen oder einen Aralkylrest mit 7 bis 20 Kohlenstoffatomen oder einen Arylrest mit 6 bis 14 Kohlenstoffatomen stehen, wobei gegebenenfalls der jeweilige Alkyl-, Cycloalkyl-, Aralkyl- oder Arylrest ein- oder mehrfach durch eine Alkyl- und/oder Alkoxygruppe mit 1 bis 6 Kohlenstoffatomen und/oder durch Fluor, Chlor, Brom und/oder Iod und/oder durch eine durch Fluor, Chlor, Brom und/oder Iod ein- oder mehrfach substituierte Alkyl- und/oder Alkoxygruppe mit 1 bis 6 Kohlenstoffatomen substituiert ist,

$R^3$ einen unverzweigten oder verzweigten, substituierten oder unsubstituierten Alkylrest mit 1 bis 28 Kohlenstoffatomen darstellt und

$R^4$ für $-OR^5$ und $-NR^5R^6$ steht, wobei $R^5$ und $R^6$ gleich oder verschieden sind und die für $R^1$ und $R^2$ angegebene Bedeutung haben,

gefunden, das dadurch gekennzeichnet ist, daß man ungesättigte Carbonsäureamide der Formel

$$R^7\text{-CO-NR}^1R^2 \qquad (II),$$

worin

$R^1$ und $R^2$ die oben angegebene Bedeutung besitzen und

$R^7$ einen α,β- oder β,γ-ungesättigten, unverzweigten oder verzweigten, substituierten oder unsubstituierten Alkylrest mit 3 bis 30 Kohlenstoffatomen darstellt,

mit H-aciden nukleophilen Verbindungen der Formel

$$HX \qquad (III),$$

worin

X für $-OR^5$ und $-NR^5R^6$ steht und $R^5$ und $R^6$ die obengenannte Bedeutung haben,

mit Kohlenmonoxid in Gegenwart von Kobaltverbindungen und gegebenenfalls in Gegenwart von einer oder mehreren tertiären Stickstoffbasen bei erhöhtem Druck und erhöhter Temperatur umsetzt.

Nach dem erfindungsgemäßen Verfahren können insbesondere mit einer hohen Selektivität unterschiedlich derivatisierte, substituierte Bernsteinsäureamide der Formel

$$R^{11}-\overset{O}{\overset{\|}{C}}-\underset{R^{10}}{\underset{|}{CH}}-CH_2-\overset{O}{\overset{\|}{C}}-NR^8R^9, \qquad (IV)$$

worin

$R^8$ und $R^9$ gleich oder verschieden sein können und die Bedeutung von $R^1$ und $R^2$ haben,

$R^{10}$ die Bedeutung von $R^3$ besitzt und

$R^{11}$ die Bedeutung von $R^4$ hat, mit der Maßgabe, daß bei einem Diamid die Amidgruppen nicht identisch sein dürfen,

erhalten werden.

Besonders bevorzugt werden unterschiedlich derivatisierte, substituierte Bernsteinsäureamide der Formeln

$$R^{12}O-\overset{O}{\overset{\|}{C}}-\underset{R^{10}}{\underset{|}{CH}}-CH_2-\overset{O}{\overset{\|}{C}}-NR^8R^9, \qquad (V)$$

und

$$R^{12}R^{13}N-\overset{O}{\overset{\|}{C}}-\underset{R^{10}}{\underset{|}{CH}}-CH_2-\overset{O}{\overset{\|}{C}}-NR^8R^9, \qquad (VI)$$

worin

$R^{12}$ und $R^{13}$ gleich oder verschieden sein können und die für $R^1$ und $R^2$ angegebene Bedeutung haben und

$R^8$, $R^9$ und $R^{10}$ die obengenannte Bedeutung besitzen, mit der Maßgabe, daß in Formel VI die Amidreste $NR^{12}R^{13}$ und $NR^8R^9$ unterschiedlich sind,

erhalten.

Als α,ß- oder ß,γ-olefinisch ungesättigte, unverzweigte oder verzweigte, substituierte oder unsubstituierte Alkylreste R7 kommen insbesondere solche mit 3 bis 10 Kohlenstoffatomen in Betracht, wie Prop-1-en-1-yl, Prop-2-en-1-yl, But-1-en-1-yl, But-2-en-1-yl, Pent-1-en-1-yl, Pent-2-en-1-yl, Hex-1-en-1-yl, Hex-2-en-1-yl, Hept-1-en-1-yl, Oct-1-en-1-yl, Non-1-en-1-yl, Dec-1-en-1-yl, 2-Methylprop-1-en-1-yl, Hept-3-en-3-yl, 3-Chlor-prop-1-en-1-yl, 3-Bromprop-1-en-1-yl, 3-Phenylprop-1-en-1-yl und 3-Phenylprop-2-en-1-yl, bevorzugt Prop-1-en-1-yl, Prop-2-en-1-yl, But-1-en-1-yl, But-2-en-1-yl, Oct-1-en-1-yl, 3-Bromprop-1-en-1-yl und 3-Phenylprop-1-en-1-yl.

Als Alkylreste $R^1$ und $R^2$ der Formel (I) kommen vorzugsweise solche mit 1 bis 12 Kohlenstoffatomen in Betracht, wie der Methyl-, Ethyl-, n-Propyl-, iso-Propyl-, n-Butyl-, iso-Butyl-, 2-Ethylhexyl-, n-Heptyl- und der Decylrest, bevorzugt der Methyl-, Ethyl-, n-Propyl- und n-Butylrest, als Cycloalkylreste solche mit 6 bis 12 Kohlenstoffatomen, wie der Cyclohexyl-, Cyclooctyl- und der Cyclododecylrest, bevorzugt der Cyclohexylrest, als Aralkylreste solche mit 7 bis 12 Kohlenstoffatomen, wie der Benzyl-, 1-Phenyl-eth-1-yl- und der 2-Phenyl-eth-1-ylrest,

bevorzugt der Benzylrest und als Arylreste solche mit 6 bis 10 Kohlenstoffatomen, wie der Phenyl- oder der Naphthylrest, bevorzugt der Phenylrest.

Die Alkyl-, Cycloalkyl-, Aralkyl- und Arylreste $R^1$ und $R^2$ können noch zusätzlich durch eine oder mehrere Alkyl- und/oder Alkoxygruppen mit 1 bis 6 Kohlenstoffatomen und/oder durch Fluor, Chlor, Brom und/oder Iod und/oder durch eine durch Fluor, Chlor, Brom und/oder Iod ein- oder mehrfach substituierte Alkyl- und/oder Alkoxygruppe mit 1 bis 6 Kohlenstoffatomen, bevorzugt durch Methyl-, Ethyl-, Methoxy-, Ethoxy- und/oder Trifluormethylgruppen sowie durch Fluor und/oder Chlor, substituiert sein. Zum Beispiel seien als mögliche Reste mit solchen Substituenten genannt: 2-Methoxyethyl, 2-Ethoxyethyl, o-, m-, p-Kresyl, o-, m-, p-Chlorphenyl, o-, m-, p-Fluorphenyl, 3,5-Dimethylphenyl, 3,5-Dichlorphenyl, Pentafluorphenyl und 3,5-Bis(trifluormethyl)-phenyl.

In das erfindungsgemäße Verfahren können demnach als α,β- oder β,γ-olefinisch ungesättigte Carbonsäurederivate zum Beispiel eingesetzt werden: Crotonsäure-, Pent-2-ensäure-, Pent-3-ensäure-, Hex-2-ensäure-, Hex-3-ensäure, Non-2-ensäure-, 3-Bromcrotonsäure-, 3-Phenylcrotonsäure-methylamid, -ethylamid, -dimethylamid, -diethylamid, -diphenylamid, -benzylamid, -dibenzylamid, -N-methylbenzylamid, -N-ethylbenzylamid, -anilid, -N-methylanilid, -N-ethylanilid, -N-benzylanilid, -3,5-dichloranilid und -3,5-dimethylanilid.

Als H-acide nukleophile Verbindungen der Formel (III) können in das erfindungsgemäße Verfahren eingesetzt werden Wasser, aliphatische und aromatische Alkohole, Ammoniak sowie aliphatische und aromatische Amine. Welcher Alkohol oder welches Amin als H-acide nucleophile Komponente in das erfindungsgemäße Verfahren eingesetzt wird, hängt davon ab, welche unterschiedlich derivatisierten, in 2-Stellung substituierten Bernsteinsäuren hergestellt werden sollen. So können als gegebenenfalls durch eine Alkyl- und/oder Alkoxygruppe mit 1 bis 6 Kohlenstoffatomen und/oder durch Fluor, Chlor, Brom und/oder Iod ein- oder mehrfach substituierte aliphatische oder cycloaliphatische Alkohole, $R^5OH$, solche eingesetzt werden, deren Rest $R^5$ 1 bis 20 Kohlenstoffatome, bevorzugt 1 bis 12 Kohlenstoffatome, wie Methyl, Ethyl, n-Propyl, iso-Propyl, n-Butyl, iso-Butyl, n-Pentyl, n-Hexyl, n-Heptyl, n-Octyl, n-Oct-2-yl, n-Decyl, Cyclohexyl, Cyclooctyl und Cyclododecyl enthält.

Als einsetzbare substituierte Alkohole seien z.B. 2-Methoxyethanol, 2-Ethoxyethanol, 2-n-Butoxyethanol, 2-Chlorethanol, 1-, 2-, 3-, 4-Methylcyclohexanol und 2-Chlorcyclohexanol genannt.

Als gegebenenfalls durch eine Alkyl- und/oder Alkoxygruppe mit 1 bis 6 Kohlenstoffatomen und/oder durch Fluor, Chlor, Brom und/oder Iod ein- oder mehrfach substituierte arylaliphatische oder aromatische Alkohole, $R^3OH$, können solche eingesetzt werden, deren Rest $R^3$ 6 bis 18 Kohlenstoffatome, bevorzugt 6 bis 12 Kohlenstoffatome enthält, wie der Benzyl-, 1-Phenyl-eth-1-yl-, 2-Phenyl-eth-1-yl-, Phenyl- und Naphthylrest.

Als einsetzbare Alkohole mit substituiertem Arylrest seien z.B. genannt: o-, m-, p-Kresol, 2,3-, 2,4-, 2,5-, 2,6-, 3,5-Xylenol, o-, m-, p-Chlorphenol, o-, m-, p-Fluorphenol, 4-Methoxyphenol, 6-Brom-α-naphthol, 6-Brom-ß-naphthol, 2-Methoxy-4-n-propyl-phenol, p-Chlorbenzylalkohol, p-Fluorbenzylalkohol und Pentafluorbenzylalkohol.
Neben Ammoniak können als aliphatische oder aromatische Amine mit mindestens einem beweglichen Wasserstoffatom am Stickstoff z.B. eingesetzt werden: Methyl-, Ethyl-, n-Propyl-, iso-Propyl-, n-Butyl-, iso-Butyl-, Benzyl-, Dimethyl-, Diethyl-, n-Butylmethyl-, Dibenzyl-, Cyclohexyl-, N-Methylcyclohexyl-, N-Ethylcyclohexylamin oder Anilin oder substituierte Aniline, wie N-Methyl-, N-Ethyl-, N-Benzyl-, o-, m-, p-Chlor-, o-, m-, p-Fluor-, o-, m-, p-Methyl-, 2,4-, 2,6-, 3,5-Dimethyl-, 2,3-, 2,4-, 2,6-, 3,4-, 3,5-Dichlor-, 2,4,5-, 2,4,6-Trichlor- und 3,5-Bis(trifluormethyl)anilin. Desgleichen ist die Verwendung von Diolen und höherwertigen Alkoholen, wie Ethylenglykol, Propandiol-1.3, Butandiol-1.4, Trimethylolpropan und Pentaerythrit sowie von Diaminen und höherwertigen Aminen mit mindestens einem beweglichen Wasserstoffatom am Stickstoff wie Ethylendiamin oder Hexamethylendiamin, möglich.

Der Umsatz der als Substrate eingesetzten α,ß- oder ß,γ-ungesättigten Carbonsäureamide der Formel (II) liegt in der Regel bei etwa 60 bis 100 %. Andererseits ist es für das erfindungsgemäße Verfahren unerheblich, die Umsätze des Substrates auf Werte unter 60 % zu begrenzen.

Ferner kann es zweckmäßig sein, die Umsetzung in Gegenwart eines inerten organischen Lösungs- und/oder Verdünnungsmittels durchzuführen. Beispielsweise kann man die erfindungsgemäße Umsetzung in Gegenwart von Toluol, Tetrahydrofuran, Dioxan, Acetonitril und/oder Di-n-butylether durchführen. Die Menge des einzusetzenden inerten Lösungs- und/oder Verdünnungsmittels ist nicht kritisch und kann erforderlichenfalls durch einige Vorversuche leicht ermittelt werden.

Die einzusetzende Menge an H-acider nukleophiler Verbindung der allgemeinen Formel (II) sollte zumindestens äquimolar bezogen auf das umzusetzende ungesättigte Carbonsäureamid sein. Als günstig haben sich Mengen an nukleophiler Komponente von etwa 1,05 bis 5 Mol/Mol ungesättigtes Carbonsäureamid erwiesen.

Andererseits behindern größere Überschüsse das erfindungsgemäße Verfahren nicht, da zudem die nukleophile Komponente bei manchen Umsetzungen als Lösungs- und/oder Verdünnungsmittel Verwendung finden kann, wodurch auf den Einsatz eines systemfremden Lösungs- und/oder Verdünnungsmittels verzichtet werden kann.

Für die Herstellung der substituierten Bernsteinsäureamide werden Kohlenmonoxid-Drucke von etwa 20 bis 4000 bar angewandt. Als vorteilhaft haben sich Kohlenmonoxid-Drucke von 50 bis 1000 bar, als besonders vorteilhaft solche von 80 bis 300 bar erwiesen.

Nach dem erfindungsgemäßen Verfahren ist es zweckmäßig, das Kohlenmonoxid im Überschuß, vornehmlich in 2 bis 100

molarem Überschuß, bezogen auf das umzusetzende Substrat, einzusetzen. Überschüssiges Kohlenmonoxid kann nach der Umsetzung leicht, z.B. durch Entspannung, von den übrigen Reaktionskomponenten abgetrennt und als solches wiederum verwendet werden.

Zur Erzielung akzeptabler Reaktionsgeschwindigkeiten ist es nützlich, dem Kohlenmonoxid etwa 0,5 bis 10 Vol.-% Wasserstoff, bevorzugt 1 bis 3 Vol.-% Wasserstoff beizumischen.

Bei den als Katalysatoren eingesetzten Kobaltverbindungen handelt es sich um Carbonylkomplexe bzw. Hydrocarbonylkomplexe des Kobalts. Es können jedoch auch Chloride, Bromide, Iodide, Acetale, Oxide, Carbonate, Sulfate oder sonstige Kobaltverbindungen, aus welchen unter den gegebenen Reaktionsbedingungen katalytisch aktive Kobaltcarbonylverbindungen entstehen, verwendet werden. Andererseits ist es auch möglich, bei Einsatz von nicht-carbonylhaltigen Kobaltverbindungen einen sogenannten Präformierschritt, d.h. eine gezielte Vorbehandlung der Kobaltverbindung zur Erzeugung der katalytisch aktiven Verbindung, der eigentlichen Umsetzung voranzustellen. Bevorzugt eingesetzte Kobaltverbindung ist das Dikobaltoctacarbonyl, $Co_2(CO)_8$. Es ist zweckmäßig, die Kobaltcarbonylkomplexe in solchen Mengen einzusetzen, daß pro g-Atom Kobalt etwa 1 bis 200 Mol Substrat, bevorzugt 10 bis 100 Mol Substrat vorliegen. Besonders vorteilhaft sind Substrat:Kobalt-Verhältnisse von 15 bis 75:1.

Für das erfindungsgemäße Verfahren kann es gegebenenfalls von Vorteil sein, die Umsetzung in Gegenwart von tertiären Stickstoffbasen, vorzugsweise aromatischen tertiären Stickstoffbasen, deren $pK_A$-Wert bei etwa 3 bis 10, bevorzugt bei etwa 4 bis 9 liegt, durchzuführen. Die Gegenwart einer oder mehrerer derartiger Stickstoffbasen kann gegebenenfalls die Selektivität der substituierten Bernsteinsäureamide erhöhen. Ob eine Selektivitätsverbesserung durch Zusatz derartiger tertiärer Stickstoffbasen zu erzielen ist, läßt sich leicht in einigen Vorversuchen ermitteln.

Als Stickstoffbasen kommen insbesondere N-heterocyclische Stickstoffverbindungen, die in ortho-Stellung zum Heteroatom nicht substituiert sind, in Betracht. Beispielsweise können eingesetzt werden: Pyridin, Isochinolin, ß-Picolin, $\gamma$-Picolin, 3,5-Lutidin, 4-Ethylpyridin und/oder 4-Benzylpyridin, bevorzugt Pyridin, $\gamma$-Picolin und/oder Isochinolin.

Die einzusetzenden Basen können neben Stickstoffatomen auch andere Heteroatome, z.B. Sauerstoff oder Chlor, enthalten.

Üblicherweise werden die Stickstoffbasen, die sowohl einzeln als auch im Gemisch untereinander eingesetzt werden können, und die ungesättigten Carbonsäureamide, das Substrat, in einem Molverhältnis von etwa 0,001 : 1 bis 1:1, bevorzugt in einem Molverhältnis von etwa 0,01 :1 bis 0,2:1 eingesetzt. Höhere Basenkonzentrationen bringen wirtschaftlich keine Vorteile.

Die Reaktion wird bei einer Temperatur von etwa 90 bis 220°C, vorzugsweise von 120 bis 190°C durchgeführt. Je nachdem, welches substituiertes Bernsteinsäuramid synthetisiert werden soll, ist die Festlegung einer oberen Reaktionstemperatur sinnvoll, um Folgereaktionen des Bernsteinsäureamids, z.B. Ringschlußreaktionen, zu verhindern. Die für die jeweilige Umsetzung optimale Reaktionstemperatur läßt sich in einigen Vorversuchen leicht ermitteln.

Die Durchführung der Reaktion kann sowohl kontinuierlich als auch diskontinuierlich erfolgen.

Das erfindungsgemäße Verfahren sei am Beispiel der Umsetzung von Crotonsäurediethylamid mit Kohlenmonoxid und Methanol wie folgt formelmäßig dargestellt:

$$CH_3\text{-}CH{=}CH\text{-}\overset{\overset{O}{\|}}{C}\text{-}NEt_2 \quad + \quad CO \quad + \quad CH_3OH \quad \xrightarrow[\text{N-Base}]{\text{Co}}$$

$$H_3CO\text{-}\overset{\overset{O}{\|}}{C}\text{-}\underset{\underset{CH_3}{|}}{CH}\text{-}CH_2\text{-}\overset{\overset{O}{\|}}{C}\text{-}NEt_2$$

Das erfindungsgemäße Verfahren kann allgemein wie folgt durchgeführt werden:

Ein mit Stickstoff oder Argon gespülter Autoklav wird z.B. mit dem Alkohol, Ammoniak oder dem Amin, gegebenenfalls der oder den tertiären Stickstoffbase(n), dem Kobaltkatalysator, dem ungesättigten Carbonsäurederivat

und gegebenenfalls dem inerten Lösungs- und/oder Verdünnungsmittel beschickt.
Dann wird bei Raumtemperatur Kohlenmonoxid, welches die notwendige Menge Wasserstoff enthält, aufgepreßt, und zwar soviel, daß bei der gewünschten Reaktionstemperatur der vorgegebene Reaktionsdruck aufgebaut wird. Anschließend wird der Autoklaveninhalt auf Reaktionstemperatur aufgeheizt und unter Rühren die vorgegebene Zeit bei dieser Temperatur (± 3°C) gehalten. Der Reaktionsdruck wird während der Reaktion durch sukzessive Nachspeisung des Reaktionsgases in einem Bereich von ± 5 bar konstant gehalten. Anschließend wird das Produktgemisch abgekühlt, der Autoklav entspannt und das erhaltene Reaktionsgemisch, je nach Erfordernis nach Zusatz eines Lösungsmittels, gaschromatographisch analysiert. Das Produktgemisch kann durch Destillation bzw. Kristallisation in jeweils üblicher Weise aufgearbeitet werden.

Nach dem erfindungsgemäßen Verfahren lassen sich z.B. 2-Alkylbernsteinsäure-1-alkylester-4-amide, 2-Alkylbernsteinsäure-1-cycloalkylester-4-amide, 2-Alkylbernsteinsäure-1-aralkylester-4-amide, 2-Alkylbernsteinsäure-1-arylester-4-amide und 2-Alkylbersteinsäure-1-amid-4-amide (mit unterschiedlichen Amidresten) in einem Reaktionsschritt synthetisieren.

Bei den mit Hilfe des erfindungsgemäßen Verfahrens vorzugsweise synthetisierten unterschiedlich derivatisierten, substituierten Bernsteinsäureamiden handelt es sich

größtenteils um neue Verbindungen. Zum Beispiel werden genannt: 2-Methylbernsteinsäure-1-phenylester-4-diethylamid, 2-Methylbernsteinsäure-1-ethylester-4-diethylamid, 2-Methylbernsteinsäure-1-cyclohexylester-4-diethylamid, 2-Methylbernsteinsäure-1-(3,5-dichloranilid)-4-diethylamid, 2-Methylbernsteinsäure-1-N-methylanilid-4-amid, 2-Ethylbernsteinsäure-1-ethylester-4-diethylamid und 2-Methylbernsteinsäure-1-/3,5-bis(trifluormethyl)anilid7-4-diethylamid.

Ihre Charakterisierung erfolgte durch Elementaranalyse und/oder IR- und/oder Protonen-Kernresonanz-Spektrometrie.

Die nach dem erfindungsgemäßen Verfahren erhaltenen unterschiedlich derivatisierten, in 2-Stellung substituierten Bernsteinsäuren eignen sich als Antioxidantien sowie als Stabilisatoren in Polymerwerkstoffen. Desweiteren können sie als Vorprodukte für die Synthese von Insektiziden, Fungiziden, Herbiziden und von Akariziden sowie von pharmakologischen und physiologischen Wirkstoffen dienen.

Beispiel 1

In einem mit Stickstoff gespülten 0,25 l Schüttelautoklaven wurden 56,5 g Crotonsäurediethylamid, 56,5 g Phenol, 6,33 g Pyridin und 2,74 g $Co_2(CO)_8$ vorgelegt.

Nach dem Verschließen des Autoklavens wurde soviel Kohlenmonoxid, welches ca. 2 Vol.-% Wasserstoff enthielt, aufgepreßt, daß bei Erreichen der Reaktionstemperatur der Gesamtgasdruck 150 bar betrug. Anschließend wurde der Autoklav mittels einer elektrischen Heizung auf 170°C aufgeheizt und 45 Minuten unter Fortsetzung der Schüttelung bei dieser Temperatur gehalten. Verbrauchtes Reaktionsgas wurde durch sukzessive Nachspeisung von frischem Reaktionsgas ersetzt, so daß der Reaktionsdruck bei 150 bar (± 5 bar) konstant gehalten wurde. Nach dem Abkühlen des Reaktionsgemisches wurde der Autoklav entspannt und das Produktgemisch gaschromatographisch analysiert. Die Analyse zeigte, daß bezogen auf einen Umsatz an Crotonsäurediethylamid von 91,5 Mol-% 56,9 Mol-% unterschiedlich derivatisierte $C_5$-Dicarbonsäurederivate entstanden waren, wovon 87,7 % 2-Methylbernsteinsäure1-phenylester-4-diethylamid und 12,3 % Glutarsäure-phenylester-diethylamid waren.

2-Methylbernsteinsäure-1-phenylester-4-diethylamid

$Kp_{0,3}$ = 155°C $\qquad n_D^{20}$ = 1,5069

Elementaranalyse:

| | | | |
|---|---|---|---|
| gef.: | 68,23 % C, | 8,10 % H, | 5,24 % N, |
| ber.: | 68,41 % C, | 8,04 % H, | 5,32 % N, |

IR: $\nu_{C=O}$ (Ester) = 1758 $cm^{-1}$; $\nu_{C=O}$ (Amid) = 1641 $cm^{-1}$

$^{1}$H-NMR: $\delta$ = 1,15 ppm (q, 6H); $\delta$ = 1,39 ppm (d, 3H), $\delta$ = 2,57 ppm, 2,81 ppm (2d, 2H); $\delta$ = 3,14 ppm (m, 1H), $\delta$ = 3,39 ppm (m, 4H); $\delta$ = 7,28 ppm (m, 5H).

Beispiel 2

Analog Beispiel 1 werden 56,5 g Crotonsäurediethylamid, 36,9 g Ethanol, 1,27 g Pyridin und 2,74 g $Co_2(CO)_8$ 1,5 Stunden bei 170°C unter 150 bar Kohlenmonoxid (+ ca. 2 Vol.-% Wasserstoff) umgesetzt. Die Analyse ergab, daß sich 99,6 Mol-% des eingesetzten Crotonsäurediethylamids umgesetzt hatten. Darauf bezogen waren mit einer Selektivität von 97,4 Mol-% gemisch derivatisierte $C_5$-Dicarbonsäurederivate entstanden; der Anteil des 2-Methylbernsteinsäure-1-ethylester-4-diethylamids betrug davon 93,6 %. Darüber hinaus hatten sich mit Selektivitäten von 1,2 Mol-% bzw. 0,4 Mol-% Buttersäurediethylamid bzw. But-3-ensäurediethylamid gebildet.

2-Methylbernsteinsäure-1-ethylester-4-diethylamid

$Kp_{0,4}$ = 89-91°C; $n_D^{20}$ = 1,4507

$^1$H-NMR: $\delta$ = 1,14 ppm (q, 6H); $\delta$ = 1,22 ppm (d, 3H); $\delta$ = 1,26 ppm (tr, 3H); $\delta$ = 2,39 ppm, 2,68 ppm (2d, 2H); $\delta$ = 3,03 ppm (m, 1H); $\delta$ = 3,38 ppm (dq, 4H); $\delta$ = 4,13 ppm (q, 2H).

Beispiele 3 bis 6

Analog Beispiel 2 wurden die in Tabelle 1 aufgelisteten Resultate unter den gleichfalls dort aufgeführten Bedingungen erhalten. Als Substrat wurde jeweils 56,5 g (≙ 0,4 Mol) Crotonsäurediethylamid eingesetzt, die Reaktionszeit betrug jeweils 1,5 Stunden, der Reaktionsdruck jeweils 150 bar. Es werden hier und desweiteren folgende Definitionen benutzt:

$$\text{Umsatz U (Mol-\%)} = \frac{\text{Mole umgesetztes Substrat}}{\text{Mole eingesetztes Substrat}} \cdot 100\ \%$$

$$\text{Selektivität S (Mol-\%)} = \frac{\text{Mole gemische Dicarbonsäurederivate}}{\text{Mole umgesetztes Substrat}} \cdot 100\ \%$$

$I_1$ kennzeichnet den prozentualen Anteil der unterschiedlich derivatisierten, in 2-Stellung substituierten Bernsteinsäure an den insgesamt erhaltenen gemischt derivatisierten Dicarbonsäuren.

Tabelle 1

| Beispiel | molares Einsatzverh. von Substrat : | EtOH : | Pyridin : | Co | Temp. (°C) | U | S (Mol-%) | $I_1$ (%) |
|---|---|---|---|---|---|---|---|---|
| 3 | 25 | 50 | 5 | 1 | 170 | 95,3 | 92,2 | 91,5 |
| 4 | 25 | 50 | 0 | 1 | 170 | 99,5 | 96,4 | 94,0 |
| 5 | 25 | 50 | 5 | 1 | 150 | 40,7 | 63,2 | 92,6 |
| 6 | 50 | 100 | 1 | 1 | 170 | 99,6 | 83,7 | 92,1 |

Beispiel 7

70,6 g Crotonsäurediethylamid, 32,0 g Methanol, 7,9 g Pyridin und 3,42 g $Co_2(CO)_8$ wurden gemäß Beispiel 1 1,5 Stunden bei 170°C unter 150 bar CO(+ 2 % $H_2$) umgesetzt. Bei einem 58,9 Mol-%igen Umsatz an Crotonsäurediethylamid waren mit einer Selektivität von 85,6 Mol-% gemischte $C_5$-Dicarbonsäurederivate entstanden, der Anteil $I_1$ an 2-Methylbernsteinsäure-1-methylester-4-diethylamid betrug 91,5 %.

2-Methylbernsteinsäure-1-methylester-4-diethylamid

$Kp_{0,3}$ = 84°C; $n_D^{20}$ = 1,4527

$^1$H-NMR: $\delta$= 1,14 ppm (q, 6H); $\delta$= 1,23 ppm (d, 3H); $\delta$= 2,41 ppm, 2,68 ppm (2d, 2H); $\delta$= 2,99 ppm (m, 1H); $\delta$= 3,34 ppm (dq, 4H); $\delta$= 3,69 (s, 3H)

Beispiel 8

Analog Beispiel 1 wurden 56,5 g Crotonsäurediethylamid, 36,6 g Glykolmonomethylether, 1,27 g Pyridin und 2,74 g $Co_2(CO)_8$ 1 Stunde bei 170°C unter 150 bar CO ( + 2 % $H_2$) umgesetzt. Bei vollständigem Substratumsatz hatten sich 84,4 Mol-% $C_5$-Dicarbonsäure-ß-methoxyethylester-diethylamide gebildet, wovon 91,6 % 2-Methylbernsteinsäure-1-ß-methoxyethylester-4-diethylamid waren.

2-Methylbernsteinsäure-1-ß-methoxyethylester-4-diethylamid

$Kp_1$ = 125°C; $n_D^{20}$ = 1,4548

$^1$H-NMR: δ= 1,15 ppm (q, 6H); δ= 1,24 ppm (d, 3H); δ= 2,42 ppm, 2,69 ppm (2d, 2H); δ = 3,03 ppm (m, 1H); δ = 3,36 ppm (dq, 4H); δ= 3,41 ppm (s, 3H); δ= 3,60 ppm (tr, 2H); δ= 4,26 ppm (tr, 2H)

Beispiel 9

Beispiel 8 wurde wiederholt mit den Ausnahmen, daß 45,7 g Glykolmonomethylether und 6,33 g Pyridin eingesetzt und die Umsetzung 2 Stunden bei 160°C durchgeführt wurden. Dabei setzten sich 42,8 Mol-% des Crotonsäurediethylamids um. Darauf bezogen wurden 65,4 Mol-% unterschiedlich derivatisierte $C_5$-Dicarbonsäuren gebildet, der Anteil $I_1$ betrug 90,7 %.

Beispiel 10

Beispiel 8 wurde wiederholt mit den Ausnahmen, daß anstelle des Glykolmonomethylethers 48,1 g Cyclohexanol eingesetzt wurden. Die Reaktionszeit betrug 0,5 Stunden. Die Analyse zeigte, daß der Umsatz des Crotonsäurediethylamids 99,7 Mol-% betrug, die Selektivität an gemischten Dicarbonsäurederivaten 82,8 Mol-% und der Anteil $I_1$ des 2-Methylbernsteinsäure-1-cyclohexylesters-4-diethylamids 92,1 %.

2-Methylbernsteinsäure-1-cyclohexylester-4-diethylamid

$Kp_{0,4}$ = 138 °C; $n_D^{20}$ = 1,4715

$^1$H-NMR: δ = 1,15 ppm (q, 6H); δ = 1,21 ppm (d, 3H); δ = 1,62 ppm (m, 10H); δ = 2,38 ppm, 2,68 ppm (2d, 2H); δ = 3,03 ppm (m, 1H); δ = 3,35 ppm (dq, 4H); δ = 4,77 ppm (m, 1H)

Beispiel 11

Beispiel 10 wurde wiederholt mit der Ausnahme, daß anstelle des Cyclohexanols 51,9 g Benzylalkohol eingesetzt wurden. Die Reaktionstemperatur betrug 150 °C. Nach 30 Minuten hatten sich von den 98,2 Mol-% umgesetzten Crotonsäurediethylamid 73,9 Mol-% $C_5$-Dicarbonsäurebenzylester-diethylamide gebildet, wovon 92,0 Mol-% 2-Methylbernsteinsäure-1-benzylester-4-diethylamid waren.

2-Methylbernsteinsäure-1-benzylester-4-diethylamid

$Kp_{0,4}$ = 171 °C; $n_D^{20}$ = 1,5054

$^1$H-NMR: δ = 1,11 ppm (q, 6H); δ = 1,23 ppm (d, 3H); δ = 2,41 ppm, 2,69 ppm (2d, 2H); δ = 3,01 ppm (m, 1H); δ = 3,32 ppm (m, 4H); δ = 5,15 ppm (d, 2H); δ = 7,37 ppm (s, 5H)

Beispiel 12

Analog Beispiel 10 wurde Crotonsäurediethylamid mit 64,9 g p-Kresol umgesetzt. Nach 30 Minuten betrug der Substratumsatz 99,4 Mol-%, die Selektivität S an gemischten $C_5$-Dicarbonsäurederivaten 76,6 Mol-% und der Anteil $I_1$ an 2-Methylbernsteinsäure-1-p-kresylester-4-diethylamid 91,6 %.

2-Methylbernsteinsäure-1-p-kresylester-4-diethylamid

$Kp_{0,35}$ = 163°C; $n_D^{20}$ = 1,5056

$^1$H-NMR: $\delta$= 1,15 ppm (q, 6H); $\delta$= 1,38 ppm (d, 3H); $\delta$= 2,38 ppm (s, 3H); $\delta$= 2,53 ppm, 2,78 ppm (2d, 2H); $\delta$= 3,12 ppm (m, 1H); $\delta$= 3,37 ppm (m, 4H); $\delta$= 7,12 ppm (m, 4H)

Beispiel 13

42,4 g Crotonsäurediethylamid, 72,9 g 3,5-Dichloranilin, 4,75 g Pyridin, 2,05 g $Co_2(CO)_8$ und 43,2 g Tetrahydrofuran wurden gemäß Beispiel 1 75 Minuten bei 150°C unter 150 bar CO (+ ca. 2 Vol.-% $H_2$) umgesetzt.

Bei einem Umsatz an Crotonsäurediethylamid von 95,1 Mol-% hatten sich mit einer Selektivität S von 68,8 Mol-% offenkettige, gemischt derivatisierte $C_5$-Dicarbonsäuren gebildet, wovon 90,1 % 2-Methylbernsteinsäure-1-(3,5-dichlor-

anilid)-4-diethylamid waren. Als weiteres Hydrocarboxylierungsprodukt waren 8,0 Mol-% N-(3,5-Dichlorphenyl)-2-methylbernsteinsäureimid entstanden.

2-Methylbernsteinsäure-1-(3,5-dichloranilid)-4-diethylamid

F = 124 - 125°C (Petrolether/Ethanol 10:1)

$^1$H-NMR: $\delta$ = 1,14 ppm, 1,27 ppm (2tr, 6H); $\delta$ = 1,23 ppm (d, 3H); $\delta$ = 2,43 ppm, 2,87 ppm (2d, 2H); $\delta$ = 3,16 ppm (m, 1H); $\delta$ = 3,40 ppm (dq, 4H); $\delta$ = 6,92 ppm (tr, 1H); $\delta$ = 7,46 ppm (d, 2H); $\delta$ = 9,88 ppm (Offset, brs, 1H)

Beispiel 14

Beispiel 13 wurde wiederholt mit den Ausnahmen, daß 58,3 g 3,5-Dichloranilin und 0,95 g Pyridin eingesetzt und die Umsetzung bei 170°C durchgeführt wurde. Der Substratumsatz betrug 99,8 Mol-%, die Selektivitäten an $C_5$-Dicarbonsäure-3,5-dichloranilid-diethylamiden 67,8 Mol-% ($I_1$ = 85,8 %) und an N-(3,5-Dichlorphenyl)-2-methylbernsteinsäureimid 23,4 Mol-%.

Beispiel 15

Beispiel 8 wurde wiederholt, jedoch wurden anstelle des Glykolmonomethylethers 51,5 g N-Methylanilin eingesetzt.

Bei einem Crotonsäurediethylamid-Umsatz von 60,7 Mol-% wurden darauf bezogen 68,7 Mol-% gemischte $C_5$-Dicarbonsäurederivate gebildet, wovon der Anteil $I_1$ des 2-Methylbernsteinsäure-1-N-methylanilid-4-diethylamids 89,7 % betrug.

2-Methylbernsteinsäure-1-N-methylanilid-4-diethylamid

$Kp_{0,7}$ = 158 - 160°C; $n_D^{20}$ = 1,5242

$^1$H-NMR: $\delta$ = 1,05 ppm (q, 6H); $\delta$ = 1,22 ppm (d, 3H); $\delta$ = 2,19 ppm, 2,82 ppm (2d, 2H); $\delta$ = 3,04 ppm (m, 1H); $\delta$ = 3,27 ppm (s, 3H); $\delta$ = 3,32 ppm (m, 4H); $\delta$ = 7,42 ppm (m, 5H)

Beispiel 16

42,4 g Crotonsäurediethylamid, 103,1 g 3,5-Bis (trifluormethyl)-anilin, 4,75 g Pyridin, 2,05 g $Co_2(CO)_8$ und 64,9 g Tetrahydrofuran wurden gemäß Beispiel 1 2 Stunden bei 160°C unter 150 bar CO (+ ca. 2 Vol.-% $H_2$) umgesetzt.

Bei einem Umsatz an Crotonsäurediethylamid von 85,9 Mol-% hatten sich mit einer Selektivität S von 57,5 Mol-% offenkettige, gemischt derivatisierte $C_5$-Dicarbonsäuren gebildet, wovon 86,8 % 2-Methylbernsteinsäure-1-/3,5-bis(trifluormethyl)anilid/-4-diethylamid waren. Als weiteres Hydrocarboxylierungsprodukt waren 26,7 Mol-% N-/3,5-Bis(trifluormethyl)phenyl/-2-methylbernsteinsäureimid entstanden.

2-Methylbernsteinsäure-1-/3,5-bis(trifluormethyl)anilid/-4-diethylamid

F = 149 - 150°C (Petrolether/Ethanol 10:1)

$^1$H-NMR: $\delta$ = 1,14 ppm, 131 ppm (2tr, 6H); $\delta$ = 1,29 ppm (d, 3H); $\delta$ = 2,36 ppm, 3,01 ppm (2d, 2H); $\delta$ = 3,23 ppm (m, 1H); $\delta$ = 3,43 ppm (q, 4H); $\delta$ = 7,18 ppm (s, 1H); $\delta$ = 7,82 ppm (s, 2H; $\delta$ = 9,90 ppm (Offset, s, 1H).

Beispiel 17

Analog Beispiel 16 wurden 56,4 g Crotonsäurediethylamid, 137,5 g 3,5-Bis(trifluormethyl)anilin, 6,33 g Pyridin und 2,74 g $Co_2(CO)_8$ 3 Stunden bei 150°C zur Reaktion gebracht. Der Substratumsatz betrug 79,0 Mol-%, die Selektivitäten an $C_5$-Dicarbonsäure-3,5-bis(trifluormethyl)anilid-diethylamiden 26,5 Mol-% ($I_1$ = 86,8 %) und an N-/3,5-Bis(trifluormethyl)phenyl/-2-methylbernsteinsäureimid 5,1 Mol-%.

Beispiel 18

77,6 g Pent-3-ensäurediethylamid, 32,2 g Ethanol, 1,58 g Pyridin sowie 3,42 g $Co_2(CO)_8$ wurden, wie in Beispiel 1 beschrieben, 1 Stunde bei 170°C unter 150 bar Kohlenmonoxid (+ ca. 2 Vol.-% $H_2$) umgesetzt. Nach dem Abkühlen zeigte die Analyse einen Umsatz an Pent-3-ensäurediethylamid von 47,3 Mol-% an. Darauf bezogen hatten sich 61,5 Mol-% $C_6$-Dicarbonsäure-ethylester-

diethylamide gebildet, die sich wie folgt zusammensetzten: 64,6 % (= $I_1$) 2-Ethylbernsteinsäure-1-ethylester-4-diethylamid, 29,2 % 2-Methylglutarsäure-1-ethylester-5-diethylamid sowie 6,2 % Adipinsäure-ethylesterdiethylamid. Desweiteren entstanden neben Pentensäurediethylamid-Isomeren mit Selektivitäten von 1,7 Mol-% bzw. 1,1 Mol-% n-Pentansäurediethylamid bzw. $C_6$-Dicarbonsäure-bisdiethylamide (3 Isomere).

2-Ethylbernsteinsäure-1-ethylester-4-diethylamid

$Kp_1$ = 117°C; $n_D^{20}$ = 1,4516

$^1$H-NMR: $\delta$ = 0,96 ppm (tr, 3H); $\delta$ = 1,15 ppm (q, 6H); $\delta$ = 1,28 ppm (tr, 3H); $\delta$ = 1,64 ppm (m, 2H); $\delta$ = 2,42 ppm, 2,66 ppm (2d, 2H); $\delta$ = 2,92 ppm (m, 1H); $\delta$ = 3,36 ppm (q, 4H); $\delta$ = 4,16 ppm (q, 2H)

Beispiel 19

Beispiel 16 wurde wiederholt mit den Ausnahmen, daß 4,75 g Pyridin eingesetzt wurden und die Reaktionszeit 4 Stunden betrug. Es wurde ein Substratumsatz von 55,5 Mol-% erzielt; die Selektivität S an gemischten $C_6$-Dicarbonsäurederivaten lag bei 42,2 Mol-% ($I_1$ = 62,8 %).

0143303

Beispiel 20

Analog Beispiel 16 wurde die Umsetzung in Abwesenheit des Pyridins durchgeführt. Es setzten sich 99,7 Mol-% Pent-3-ensäurediethylamid um. Die Selektivität S an $C_6$-Dicarbonsäure-ethylester-diethylamiden betrug 71,5 Mol-% ($I_1$ = 62,9 %).

Beispiel 21

52,6 g Pent-3-ensäureanilid, 19,2 g Methanol, 4,75 g Pyridin und 2,05 g $Co_2(CO)_8$ wurden analog Beispiel 1 1,5 Stunden bei 170°C umgesetzt. Bei einem Pentensäureanilid-Umsatz von 58,3 Mol-% wurden mit einer Selektivität S von 37,5 Mol-% $C_6$-Dicarbonsäure-methylester-anilide gebildet ($I_1$ = 65,3 %).

Patentansprüche

1. Verfahren zur Herstellung von substituierten Bernsteinsäureamiden der Formel

$$R^4-\overset{O}{\overset{\|}{C}}-\underset{R^3}{\underset{|}{CH}}-CH_2-\overset{O}{\overset{\|}{C}}-NR^1R^2 \quad ,$$

worin

$R^1$ und $R^2$ gleich oder verschieden sein können und für Wasserstoff, einen Alkyl- oder Cycloalkyl-Rest mit 1 bis 20 Kohlenstoffatomen oder einen Aralkylrest mit 7 bis 20 Kohlenstoffatomen oder einen Arylrest mit 6 bis 14 Kohlenstoffatomen stehen, wobei gegebenenfalls der jeweilige Alkyl-, Cycloalkyl-, Aralkyl- oder Arylrest ein- oder mehrfach durch eine Alkyl- und/oder Alkoxygruppe mit 1 bis 6 Kohlenstoffatomen und/oder durch Fluor, Chlor, Brom und/oder Iod und/oder durch Fluor, Chlor, Brom und/oder Iod ein- oder mehrfach substituierte Alkyl- und/oder Alkoxygruppe mit 1 bis 6 Kohlenstoffatomen substituiert ist,

$R^3$ einen unverzweigten oder verzweigten, substituierten oder unsubstituierten Alkylrest mit 1 bis 28 Kohlenstoffatomen darstellt und

$R^4$ für $-OR^5$ und $-NR^5R^6$ steht, wobei $R^5$ und $R^6$ gleich oder verschieden sind und die für $R^1$ und $R^2$ angegebene Bedeutung haben,

dadurch gekennzeichnet, daß man ungesättigte Carbonsäureamide der Formel

$$R^7-CO-NR^1R^2 \quad ,$$

worin

$R^1$ und $R^2$ die oben angegebene Bedeutung besitzen und

$R^7$ einen α,ß- oder ß,γ-ungesättigten, unverzweigten oder verzweigten, substituierten oder unsubstituierten Alkylrest mit 3 bis 30 Kohlenstoffatomen darstellt,

mit H-aciden nukleophilen Verbindungen der Formel

$$HX \quad ,$$

worin

X für $-OR^5$ und $-NR^5R^6$ steht und $R^5$ und $R^6$ die obengenannte Bedeutung haben,

mit Kohlenmonoxid in Gegenwart von Kobaltverbindungen und gegebenenfalls in Gegenwart von einer oder mehreren tertiären Stickstoffbasen bei erhöhtem Druck und erhöhter Temperatur umsetzt.

2. Verfahren nach Anspruch 1, dadurch gekennzeichnet, daß man als H-acide nukleophile Verbindungen Wasser, Methanol, Ethanol, n-Propanol, iso-Propanol, n-Butanol, iso-Butanol, n-Pentanol, n-Hexanol, n-Heptanol, n-Octanol, n-Octan-2-ol, n-Decanol, Cyclohexanol, Cyclooctanol, Cyclododecanol, 2-Methoxyethanol, 2-Ethoxyethanol, 2-n-Butoxyethanol, 2-Chlorethanol, 1-, 2-, 3-, 4-Methylcyclohexanol, 2-Chlorcyclohexanol, 1-Phenylethanol, 2-Phenylethanol, Phenol, α-, ß-Naphthol, o-, m-, p-Kresol, 2,3-, 2,4-, 2,5-, 2,6-, 3,5-Xylenol, o-, m-, p-Chlorphenol, o-, m-, p-Fluorphenol, 4-Methoxyphenol, 6-Brom-α-naphthol, 6-Brom-ß-naphthol, 2-Methoxy-4-n-propylphenol, p-Chlorbenzylalkohol, p-Fluorbenzylalkohol, Pentafluorbenzylalkohol, Ethylenglykol, Propandiol-1,3, Butandiol-1,4, Trimethylolpropan, Pentaerythrit, Ammoniak, Methylamin, Ethylamin, n-Propylamin, iso-Propylamin, n-Butylamin, iso-Butylamin, Benzylamin, Dimethylamin, Diethylamin, n-Butylmethylamin, Dibenzylamin, Cyclohexylamin, N-Methylcyclohexylamin, N-Ethylcyclohexylamin, Anilin, N-Methylanilin, N-Ethylanilin, N-Benzylanilin, o-, m-, p-Chloranilin, o-, m-, p-Fluoranilin, o-, m-, p-Methylanilin, 2,4-, 2,6-, 3,5-Dimethylanilin, 2,3-, 2,4-, 2,5-, 3,4-, 3,5-Dichloranilin, 2,4,5-, 2,4,6-Trichloranilin, 3,5-Bis-(trifluormethyl)-anilin, Ethylendiamin oder Hexamethylendiamin einsetzt.

0143303

3. Verfahren nach den Ansprüchen 1 und 2, dadurch gekennzeichnet, daß man die H-acide nukleophile Verbindung in mindestens äquimolaren Mengen, bezogen auf das umzusetzende ungesättigte Carbonsäureamid einsetzt.

4. Verfahren nach den Ansprüchen 1 bis 3, dadurch gekennzeichnet, daß man die H-acide nukleophile Verbindung in einer Menge von 1,05 bis 5 Mol pro Mol umzusetzendes ungesättigtes Carbonsäureamid einsetzt.